# EUROPEAN PATENT APPLICATION

(11) **EP 4 501 961 A1**
(43) Date of publication of application: **05.02.2025**
(21) Application number: 22934557.4
(22) Date of filing: 21.06.2022
(51) Int. Cl.: C07K 16/28, C12N 15/13, C12N 5/20, G01N 33/577, G01N 33/574

(54) **MONOCLONAL ANTIBODY AGAINST HLA-G1, -G2, -G5 AND HLA-G6 ISOMERIC MOLECULES AND USE THEREOF**

(30) Priority: 31.03.2022 CN 202210336592
(71) Applicant: Taizhou Enze Medical Center (Group), Taizhou, Zhejiang 317099 (CN)
(72) Inventor: YAN, Weihua, Zhejiang 317099 (CN)
(74) Representative: Kransell & Wennborg KB
(86) International application number: PCT/CN2022/100233
(87) International publication number: WO 2023/184734

(57) **Abstract**

Disclosed are a monoclonal antibody (YWHG-3) against HLA-G1, -G2, -G5 and HLA-G6 isoforms and use thereof. The antibody (YWHG-3) is produced by the hybridoma deposited under CCTCC NO:202121 using an antigenic peptide of a sequence of amino acids which are common to HLA-G isoforms (HLA-G1, HLA-G2, HLA-G5 and HLA-G6) and are located at positions 209-226 in the heavy chain α3 domain of the HLA-G molecules, as an immunogen. The present invention provides a nucleotide encoding the YWHG-3 antibody of the present invention and an encoded amino acid sequence thereof. The present invention further provides use of the YWHG-3 antibody for immunohistochemistry, immunoblotting, flow cytometry and other assays for HLA-G isometric molecules (HLA-G1, HLA-G2, HLA-G5 and HLA-G6).

## Description

### FIELD OF THE INVENTION

The present invention belongs to the field of biomedicine, and relates to the following aspects of an antibody (YWHG-3) against HLA-G isoforms (HLA-G1, HLA-G2, HLA-G5 and HLA-G6): preparation of a monoclonal antibody (YWHG-3) against HLA-G isoforms (HLA-G1, HLA-G2, HLA-G5 and HLA-G6) by using an antigenic peptide of a sequence of amino acids (KTHVTHHPVFDYEATLRC) which are common to HLA-G isoforms (HLA-G1, HLA-G2, HLA-G5 and HLA-G6) and are located at positions 209-226 in the heavy chain α3 domain of the HLA-G molecules as an immunogen; a nucleotide encoding the YWHG-3 antibody of the present invention and an encoded amino acid sequence thereof; and use of the antibody (YWHG-3) for immunohistochemistry, immunoblotting, flow cytometry and other assays.

### BACKGROUND OF THE INVENTION

The human leukocyte antigen-G (HLA-G) gene, with a full length of 6.0 kb, is located at 6p21.3, the distal short arm of human chromosome 6. During protein translation, exon 1 of HLA-G mRNA encodes the signal peptide, exons 2, 3, and 4 encode the α1, α2, and α3 domains of the extracellular region, respectively, and exon 5 encodes a transmembrane region; exon 6 encodes the intracellular domain of HLA-G molecule containing only 6 amino acid residues; because exon 6 contains a stop codon, exon 7 is not transcribed; exon 8 corresponds to the 3'UTR of the HLA-G gene. The initial transcript of HLA-G is alternatively spliced to produce 7 types of mature mRNAs, which encode 7 isoforms with different molecular weights (HLA-G1, -G2, -G3, -G4, - G5, -G6 and HLA -G7). Among them, HLA-G1, HLA-G2, HLA-G3 and HLA-G4 contain a transmembrane region and are membrane-bound isoforms; HLA-G5, HLA-G6 and HLA-G7 lack transmembrane structures and are soluble isomers. The molecular weights of HLA-G1~-G7 isoforms are 39 kD, 31 kD, 22 kD, 30 kD, 34 kD, 23 kD and 16 kD, respectively.

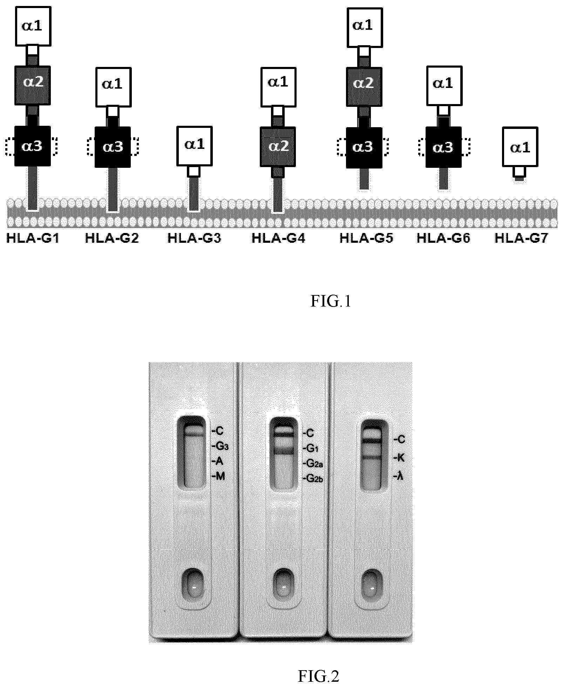

HLA-G1 is encoded by full-length HLA-G mRNA and consists of extracellular α1, α2 and α3 domains, a transmembrane region and an intracellular domain. HLA-G2 lacks α2 domain and consists of extracellular α1 and α3 domains, a transmembrane region and an intracellular domain; HLA-G3 lacks α2 and α3 domains and consists of an extracellular α1 domain, a transmembrane region and an intracellular domain; HLA-G4 lacks α3 domain and consists of extracellular α1 and α2 domains, a transmembrane region and an intracellular domain; and the extracellular domain is the same as HLA-G1 and HLA-G2 respectively. However, they are encoded by HLA-G mRNA containing intron 4. Because intron 4 contains a stop codon, the encoded protein molecules lack the transmembrane region encoded by exon 5, forming soluble HLA-G molecules. Since the mRNA encoding HLA-G7 contains a stop codon in intron 2, the extracellular region only contains the α1 domain and is connected by the two amino acid residues encoded by intron 2 **(****FIG. 1****).**

Under normal physiological conditions, HLA-G molecules are only expressed in extravillous trophoblast cells at the maternal-fetal interface, maintaining maternal-fetal immune tolerance during pregnancy. Under pathological conditions, HLA-G molecules can be inducibly expressed in tumor cells and virus-infected tissues and cells, and are closely related to the occurrence and progression of the disease. The HLA-G molecule is an important immunotoleran molecule and an important immune checkpoint molecule. Its immunosuppressive function is to transmit inhibitory signals and induce immune tolerance primarily through binding to the immunoinhibitory receptor immunoglobulin-like transcript-2, ILT2/LILRB1/CD85j) and immunoglobulin-like transcript-4 (ILT4/LILRB2/CD85d). The major mechanisms of immunosuppression induced by HLA-G/ILT signaling are as follows: ① bind to ILT-2 expressed on T cells, NK cells and B cells, inhibit the immune killing activity of T cells and NK cells, and inhibit the proliferation of B cells and the secretion of antibodies; ② bind to ILT-2 and ILT-4 expressed on dendritic cells (DC), inhibit the maturation and differentiation of DC cells, and induce the generation of tolerant DC cells; ③ bind to ILT-2 and ILT-4 expressed on myeloid-derived suppressor cells (MDSC) and macrophages (Mφ), induce the differentiation of pro-inflammatory and anti-tumor M1 macrophages into immune-resistant M2 macrophages, etc. Therefore, HLA-G plays an important role in the development of tumors and other diseases. A number of tumor immune targeted therapies targeting HLA-G have been underwent phase I or II clinical trials in the countries such as the United States.

ILT2 and ILT4 engagement with HLA-G has the HLA-G isoform molecular structure specificity. The site where receptors ILT-2 and ILT-4 bind to HLA-G is the HLA-G extracellular α3 domain. ILT-2 only binds to the HLA-G/β₂m complex, while ILT-4 can not only bind to HLA-G/β₂m, but also bind to free HLA-G molecules that do not contain β₂m. Due to differences in expression mechanism and molecular structure of HLA-G1, -G2, -G3, -G4, -G5, -G6 and HLA-G7 isoforms , ILT-2 can bind to HLA-G1 and HLA-G5, while ILT-4 can bind to HLA-G1, HLA-G2, HLA-G5 and HLA-G6 isoforms. For example, the extracellular regions of HLA-G3, -G4, and HLA-G7 isoform do not contain α3 domain and cannot bind to ILT-2 and ILT-4. Different HLA-G isoforms can exert specific immunological effects in the pathophysiological processes. Under different pathological conditions, especially in tumor tissues, there is extensive heterogeneity in the expression of HLA-G isoforms, and the expression of different isoforms of HLA-G molecules has specific clinical significance. Therefore, analyzing the expression of specific HLA-G isoforms and the expression profiles of different HLA-G isoforms is of great significance for elucidating the biological functions and clinical significance of specific HLA-G isoforms.

Currently, the available antibodies used for HLA-G immunohistochemistry and immunoblotting mainly include 4H84, MEM-G1, MEM-G2, 2A12 and 5A6G7. The antibody 4H84, whose recognition site is located in the extracellular region α1 domain that is shared by all 7 HLA-G available, can detect all 7 HLA-G isoforms (HLA-G1, HLA-G2, HLA-G3, HLA-G4, HLA-G5, HLA-G6 and HLA-G7) that are currently known to contain α1 domain, but the expression of specific HLA-G isoforms cannot be distinguished in immunohistochemistry. Antibodies MEM-G1 and MEM-G2 are obtained from mice immunized with the full-length HLA-G heavy chain, and the specific recognition sites cannot be predicted. Theoretically, the two antibodies are similar to antibody 4H84 and can recognize all 7 HLA-G isoforms (HLA-G1, HLA-G2, HLA-G3, HLA-G4, HLA-G5, HLA-G6 and HLA-G7), but cannot distinguish the expression of specific HLA-G isoforms. Antibodies 2A12 and 5A6G7 are obtained from mice immunized with the 22 amino acid residues located at the carboxyl terminus of HLA-G5 and HLA-G6 molecules, and can recognize HLA-G5 and HLA-G6 molecules. However, antibodies 2A12 and 5A6G7 can also recognize other unknown non-HLA-G5 and HLA-G6 molecules with molecular weights in the range of 36-175 kDa. When they are applied to immunohistochemistry, cross-reactions may be present and false positives may occur. The monoclonal antibodies used for flow cytometry to detect HLA-G molecules include 87G and MEM-G/9, but they can only detect HLA-G1 and HLA-G5 molecules.

Currently, there are no antibodies that can detect HLA-G1, HLA-G2, HLA-G5 and HLA-G6 isoforms.

Therefore, in the context of targeted immunotherapy and precision medicine, the existing antibodies for detecting HLA-G molecules are not satisfactory, and there is an urgent need to develop more monoclonal antibodies targeting different HLA-G isoforms.

### SUMMARY OF THE INVENTION

In view of the above shortcomings of the existing HLA-G antibodies, and there are currently no antibodies for detecting HLA-G1, HLA-G2, HLA-G5 and HLA-G6 isoforms, an object of the present invention is to provide a specific monoclonal antibody (YWHG-3) against HLA-G isoforms containing α3 domain (HLA-G1, HLA-G2, HLA-G5 and HLA-G6), a nucleotide sequence encoding the antibody (YWHG-3) of the present invention and an encoded amino acid sequence thereof; and use of the YWHG-3 antibody for immunohistochemistry, immunoblotting, flow cytometry and other assays.

According to the present invention, an antigenic peptide of a sequence of amino acids which are common to HLA-G isoformss (HLA-G1, HLA-G2, HLA-G5 and HLA-G6) and are located at positions 209-226 in the heavy chain α3 domain of the HLA-G molecules is used as an immnogen, and its amino acid sequence is the amino acid sequence (KTHVTHHPVFDYEATLRC) shown in SEQ ID No. 19. The KTHVTFHHPVFDYEATLRC peptide fragment is located in the heavy chain α3 domain of the HLA-G molecules (the area shown in the dashed box in FIG. 1), and based on this, a specific monoclonal antibody (YWHG-3) against HLA-G isoforms (HLA-G1, HLA-G2, HLA-G5 and HLA-G6) is prepared.

According to the inventor's research results, the present invention provides a monoclonal antibody (YWHG-3) against HLA-G molecules, including at least one or more of light chain hypervariable regions CDR1, CDR2 and CDR3, or/and one or more of heavy chain hypervariable regions CDR1, CDR2 and CDR3;
an amino acid sequence of the antibody light chain of the monoclonal antibody (YWHG-3) is shown in SEQ ID No. 1 or an amino acid sequence with equivalent functions to the sequence shown in SEQ ID No. 1 formed by substituting, deleting or adding one or more amino acids; an amino acid sequence of the hypervariable region CDR1 of the antibody light chain is the sequence QSLVHSTGNTF shown in SEQ ID No. 2 or an amino acid sequence with equivalent functions to the sequence shown in SEQID No. 2 formed by substituting, deleting or adding one or more amino acids; an amino acid sequence of the hypervariable region CDR2 of the light chain is the sequence KVS shown in SEQ ID No. 3 or an amino acid sequence with equivalent functions to the sequence shown in SEQ ID No. 3 formed by substituting, deleting or adding one or more amino acids, and an amino acid sequence of the hypervariable region CDR3 of the light chain is the sequence SQSTHVPWT shown in SEQ ID No. 4 or an amino acid sequence with equivalent functions to the sequence shown in SEQ ID No. 4 formed by substituting, deleting or adding one or more amino acids;
an amino acid sequence of the antibody heavy chain of the monoclonal antibody (YWHG-3) is shown in SEQ ID No. 5 or an amino acid sequence with equivalent functions to the sequence shown in SEQ ID No. 5 formed by substituting, deleting or adding one or more amino acids; an amino acid sequence of the hypervariable region CDR1 of the antibody heavy chain is the sequence GFTFSDYN shown in SEQ ID No. 6 or an amino acid sequence with equivalent functions to the sequence shown in SEQID No. 6 formed by substituting, deleting or adding one or more amino acids; an amino acid sequence of the hypervariable region CDR2 of the heavy chain is the sequence ISDGGRST shown in SEQ ID No. 7 or an amino acid sequence with equivalent functions to the sequence shown in SEQ ID No. 7 formed by substituting, deleting or adding one or more amino acids, and an amino acid sequence of the hypervariable region CDR3 of the heavy chain is the sequence VRDDY shown in SEQ ID No. 8 or an amino acid sequence with equivalent functions to the sequence shown in SEQ ID No. 8 formed by substituting, deleting or adding one or more amino acids.

Further, the monoclonal antibody (YWHG-3) also includes a light chain framework region (FR) and a heavy chain framework region; wherein, the light chain framework region comprises one or more of light chains FR1, FR2, FR3 and FR4, and an amino acid sequence of the light chain FR1 is the sequence DIMMTQTPLSLPVSLGDQASISCRSS shown in SEQ ID No. 9 or an amino acid sequence with equivalent functions to the sequence shown in SEQ ID No. 9 formed by substituting, deleting or adding one or more amino acids; an amino acid sequence of the light chain FR2 is the sequence LNWYLQKPGQSPKLLIY shown in SEQ ID No. 10 or an amino acid sequence with equivalent functions to the sequence shown in SEQ ID No. 10 formed by substituting, deleting or adding one or more amino acids; an amino acid sequence of the light chain FR3 is the sequence NRFSGVSDRFSGSGSGTDFTLKIRRVEAEDLGVYFC shown in SEQ ID No. 11 or an amino acid sequence with equivalent functions to the sequence shown in SEQ ID No. 11 formed by substituting, deleting or adding one or more amino acids; an amino acid sequence of the light chain FR4 is the sequence FGGGTKLEIK shown in SEQ ID No. 12 or an amino acid sequence with equivalent functions to the sequence shown in SEQ ID No. 12 formed by substituting, deleting or adding one or more amino acids; the heavy chain framework region comprises one or more of the heavy chains FR1, FR2, FR3 and FR4, wherein, an amino acid sequence of the heavy chain FR1 is the sequence LVQLQQSGGGLVKPGGSLKLSCAAS shown in SEQ ID No. 13 or an amino acid sequence with equivalent functions to the sequence shown in SEQ ID No. 13 formed by substituting, deleting or adding one or more amino acids; an amino acid sequence of the heavy chain FR2 is the sequence MYWVRQTPEKRLEWVAT shown in SEQ ID No. 14 or an amino acid sequence with equivalent functions to the sequence shown in SEQ ID No. 14 formed by substituting, deleting or adding one or more amino acids; an amino acid sequence of the heavy chain FR3 is the sequence FYLDSVKGRFTISRDNAKNNLYLQINNLKSEDTAIYYC shown in SEQ ID No. 15 or an amino acid sequence with equivalent functions to the sequence shown in SEQ ID No. 15 formed by substituting, deleting or adding one or more amino acids; an amino acid sequence of the heavy chain FR4 is the sequence WGQGTTLTVSS shown in SEQ ID No. 16 or an amino acid sequence with equivalent functions to the sequence shown in SEQ ID No. 16 formed by substituting, deleting or adding one or more amino acids.

Further, a nucleotide sequence encoding the light chain variable region in the monoclonal antibody (YWHG-3) is a sequence shown in SEQ ID No. 17 or a nucleotide sequence with equivalent functions to the sequence shown in SEQ ID No. 17 formed by substituting, deleting or adding one or more nucleotides; a nucleotide sequence encoding the heavy chain variable region in the monoclonal antibody (YWHG-3) is a sequence shown in SEQ ID No. 18 or a nucleotide sequence with equivalent functions to the sequence shown in SEQ ID No. 18 formed by substituting, deleting or adding one or more nucleotides.

According to one aspect of the present invention, the present invention provides a preferred monoclonal antibody (YWHG-3) against HLA-G isoforms (HLA-G1, HLA-G2, HLA-G5 and HLA-G6). The monoclonal antibody (YWHG-3) is produced by the hybridoma deposited under CCTCC NO: 202121, the depositary institution is China Center for Type Culture Collection (CCTCC) and the depositary time is August 11, 2021. The address of the CCTCC is located inside Wuhan University, Wuhan, Hubei Province, P. R. China, postal code 430072.

The present invention further provides use of the antibody (YWHG-3) against HLA-G isoforms (HLA-G1 , HLA-G2 , HLA-G5 and HLA-G6) for immunohistochemistry, immunoblotting and other assays of HLA-G isoforms (HLA-G1, HLA-G2, HLA-G5 and HLA-G6), which has the characteristics of high specificity and strong affinity, etc.

In order to understand the inventive concepts and technical solutions of the present application more clearly, the present application will be further described in detail in conjunction with specific examples and accompanying drawings. The technical solutions in the embodiments are only preferred embodiments and should not be construed as limiting the scope of the present application. For those skilled in the art, several improvements and modifications can be made without departing from the technical principles of the present application, and these improvements and modifications shall fall within the scope of protection of the present application.

Unless otherwise defined, all technical and scientific terms used herein shall be deemed to have the same meaning as commonly understood by one of ordinary skill in the art. Abbreviations for amino acid residues are the standard 3-letter and/or 1-letter codes used in the art to refer to the 20 commonly used amino acids.

For the light chain hypervariable region or heavy chain hypervariable region as mentioned herein, a "hypervariable region" is also called as a complementarity determining region (CDR).

A "sequence" mentioned in the present invention may refer to an amino acid sequences or "a conservative substitution" that contains certain biological functions; and "other sequences" may include amino acids that are not functionally equivalent or "non-conservative substitutions", which have been genetically engineered to improve the properties of CDRs or CDR-containing antibodies. Without substantially affecting the activity of the antibody, those skilled in the art can operate on the sequences in this application, i.e., substitute, add and/or delete one or more (for example, 1, 2, 3, 4, 5, 6 , 7, 8, 9 or 10 or more) amino acids to obtain a variant of the antibody or a functional fragment sequence thereof. They should be considered included in the scope of protection of the present application. For example, amino acids with similar properties are substituted in the variable region. The sequence of the variant mentioned in the present invention may be at least 95%, 96%, 97%, 98% or 99% identical to the source sequence thereof. The sequence identity can be measured using sequence analysis software. For example, the computer program BLAST with default parameters, especially BLASTP or TBLASTN, can be used. The multiple amino acid sequences described herein are detailed in the sequence listing.

### BRIEF DESCRIPTION OF THE DRAWINGS

FIG. 1 shows a structural model diagram of 7 different HLA-G isoforms and the positions of the immunogenic peptide fragments.
FIG. 2 shows the identification of isotypes of heavy chain and light chain of the antibody (YWHG-3).
FIG. 3 shows the detection of purity of the antibody (YWHG-3) by SDS-PAGE.
FIG. 4 shows the measurement of the affinity constant of the antibody (YWHG-3) by ELISA.
FIG. 5 shows the detection of HLA-G5 molecule concentration of the antibody (YWHG-3) by ELISA.
FIG. 6 shows the detection of HLA-G1, -G2, -G5 and HLA-G6 molecules of the antibody (YWHG-3) by immunoblotting.
FIG. 7 shows the detection of intracellular HLA-G1, -G2, -G5 and HLA-G6 molecules of the antibody (YWHG-3) by flow cytometry.
FIG. 8 shows use of the antibody (YWHG-3) for immunohistochemistry to detect the expressions of HLA-G1, -G2, -G5 and HLA-G6 molecules in gastric cancer tissues.

### DETAILED DESCRIPTION

### 1. Preparation of monoclonal antibody (YWHG-3) against HLA-G

### ① Synthesis of antigenic peptide

The antigenic peptide located at positions 209-226 in the heavy chain α3 domain of the HLA-G molecules was synthesized, with the SEQ No.19 KTHVTHHPVFDYEATLRC.

### ② Immunization of mice

Four SPF-grade BALB/c female mice were immunized initially, 60 ug/mouse. The dose of the first booster immunization was 30ug/mouse, the dose of the second booster immunization was 30ug/mouse, and the dose of the third booster immunization was 30ug/mouse. Blood was taken from the orbits to measure the serum titer. The plate was coated with "SEQ No.19 KTHVTHHPVFDYEATLRC", and the titer of immunized mice was determined by ELISA. The "SEQ No.19 KTHVTHHPVFDYEATLRC" was used for coating at 2 ug/ml and 4°C overnight; blocked at 37°C for 2 hours; the serum was diluted by 2-fold starting from 200-fold, and the blank control (blank) was PBS, the negative control (negative) was negative serum diluted by 200-fold. Before fusion, the "SEQ No. 19 KTHVTTHHPVFDYEATLRC" immunogen 50 ug was used to shock immunized mice. In the fusion experiment, mouse spleen cells and SP2/0 cells were taken and fused using the PEG method. After fusion, cells were screened and cultured in a semi-solid medium (containing HAT).

### ③ Screening of monoclonal cells

Cell monoclines (10 plates × 93 cells) were picked and cultured in a 96-well cell culture plate (previously plated with thymocytes, 100ul/well). The plate was coated with "SEQ No.19 KTHVTHHPVFDYEATLRC" and the "SEQ No.19 KTHVTHHPVFDYEATLRC" was diluted with the coating buffer, with the final concentration of 2 ug/ml, 100 ul/well, and cultured overnight at 4°C; then washed 3 times with a wash buffer, blocked with 2% milk blocking buffer, 200 ul/well, incubated at 37°C for 2 hours; then washed 3 times with a wash buffer. The primary antibody (cell culture supernatant), the negative control (SP2/0 culture supernatant), the blank control (PBS), and the positive control (positive serum diluted with PBS by 1000-fold) were added, 100 ul/well, incubated in a 37°C incubator for 1 hour; later washed 3 times with the wash buffer. The secondary antibody diluted with PBS by 20,000-fold was added, 100 ul/well, and incubated in a 37°C incubator for 1 hour; taken out and washed 3 times with a wash buffer. Then the color development was performed with a developing solution of 100 ul/well for about 5 minutes. Then 50 ul of stop solution was added to each well to stop. The absorbance value was measured at dual wavelengths (450, 630). The selected clones were screened using ELISA for the first time to obtain positive hybridoma cell lines. The positive cell lines were coated again with "SEQ No. 19 KTHVTHHPVFDYEATLRC", and the ELISA method was used for a second screening to obtain positive hybridoma cell lines. After multiple screenings, a monoclonal antibody against SEQ No.19 KTHVTHHPVFDYEATLRC fragment was obtained, named YWHG-3 **(deposit number: CCTCC NO: 202121).**

### ④ Isotyping of the monoclonal antibody (YWHG-3) against HLA-G

The isotyping was performed on the screened 10 positive cell lines. The coated antibody was diluted to 0.5 ug/ml with 100 mM PBS (pH 7.4), 0.1 ml was added to each well at 4°C overnight; washed twice with PBS-T, then 200 ul blocking buffer was added to each well, and incubated at 37°C for 2 hours; washed three times with PBS-T; 100 ul hybridoma supernatant was added to each well, and incubated at 37°C for 1 hour; washed three times with PBS-T; 0.1 ml of HRP-labeled antibody that was diluted with blocking buffer 1:10000 (κ, λ) or 1:20000 (others) was added to each well respectively, and incubated at 37°C for 1 hour; washed three times with PBS-T; 50 ul of substrate solution was added to each well, and then the absorbance was measured at dual wavelengths (450, 630) within 10-20 minutes. The Thermo's mouse antibody isotype rapid identification method (Pierce Rapid Isotyping Kits - Mouse, Catalog" #26178) was used to determine the isotype of the monoclonal antibody produced by the cell line to be of IgG1 type heavy chain and kappa (κ) type light chain **(****FIG. 2****).**

### ⑤ Antibody purification

Sample pretreatment: the sample was diluted by 1:3 with the corresponding coupling buffer, centrifuged at 12,000 rpm for 10 minutes at 4°C, and filtered with a 0.22 µm filter membrane to remove the fat, cell debris, and small particulate matter. Equilibration: the column was equilibrated with the corresponding coupling buffer equivalent to 10-fold column volume, and the flow rate was maintained at 1 ml/min. Sample loading: The sample was injected to the upper port of the column, and the effluent was collected, the flow rate was maintained at 1 ml/min. Impurity washing: the column was washed with the coupling buffer equivalent to 5-fold column volume, and the flow rate was maintained at 1 ml/min. Elution: The antibody was eluted with the elution buffer equivalent to 5-fold column volume, and then collected in the above EP tubes; the flow rate was maintained at 1 ml/min. The pH was adjusted to 7.0 with 1 M Tris-Hcl buffer immediately. Equilibration: the column was equilibrated with the coupling buffer equivalent to 10-fold column volume to pH 7.0, and the flow rate was maintained at 1 ml/min. Dialysis: the antibody was dialyzed overnight with 0.01 M PBS buffer, and the fluid was changed for 3 times.

### ⑥ Detection of antibody purity by SDS-PAGE

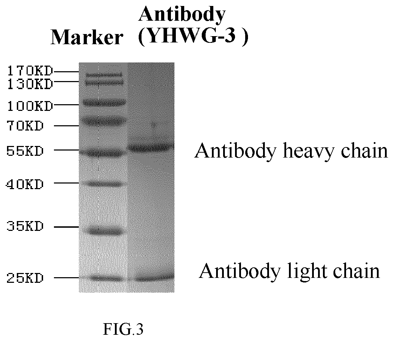

The SDS-PAGE gel was prepared, with the concentration of the separating gel of 12%. Sample preparation: the sample was added a loading buffer and boiled in boiling water for 10 minutes. Sample loading: 10 ul per well. Gel separation: 80 V for stacking gel, 30 min; and 120 V for separating gel, 60 min. When the front bromophenol blue reached the bottom of the glass plate, the electrophoresis was stopped and the gel was taken out. Staining and destaining: the gel was immersed in Coomassie Brilliant Blue staining solution and slowly shaken on a shaker for more than 30 minutes (the staining time was adjusted appropriately according to the thickness of the gel). The gel was taken out and rinsed in water for several times, and then Coomassie Brilliant Blue destaining solution was added and shaken. It would take about 1 hour when the gel was destained to see the bands, for complete destaining, the destaining solution should be replaced 2-3 times and shaken for more than 24 hours. After destaining, the gel was scanned and recorded by the ECL gel imaging system. The purity of the purified antibody YWHG-3 was >90% (FIG. 3).

### ⑦ Measurement of affinity constant of the antibody by ELISA

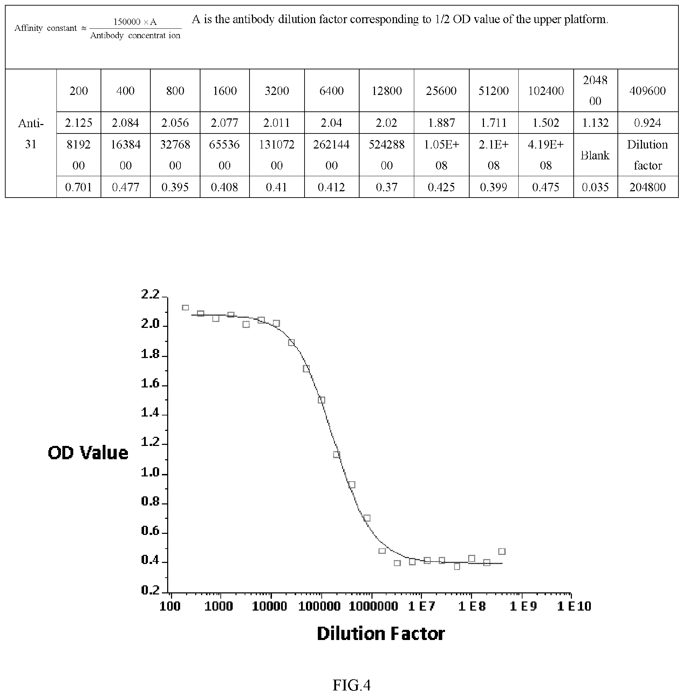

The antigen (SEQ No. 19 KTHVTHPVFDYEATLRC) was diluted with the coating buffer, with the final concentration of 2 ug/ml, 100 ul/well, and placed at 4°C overnight; washed with the wash buffer twice, blocked with the blocking buffer at 200 ul/well, incubated at 37°C for 2 hours; then washed once with the wash buffer. The purified antibody was diluted by 2-fold starting from 200-fold (PBS), and the blank control (blank) was PBS, 100 ul/well, and incubated at 37°C for 1 hour; and then washed 3 times with the wash buffer. The secondary antibody diluted with PBS by 20,000-fold was added, 100 ul/well, and incubated in a 37°C incubator for 1 hour; taken out and washed 3 times with a wash buffer. Then the color development was performed with a developing solution of 100 ul/well for 5-15 minutes. Then 50 ul of stop solution was added to each well to stop. The absorbance value was measured at dual wavelengths (450, 630), and a chart was drawn for analysis. According to the Logistic fitting equation, the affinity constant was approximately equivalent to 150000×A/antibody concentration (A was the antibody dilution factor corresponding to 1/2 OD value; dilution factor=204800; concentration of YWHG-3 antibody =2.8 mg/mL), and the affinity constant of the antibody YWHG-3 was 1.10×10¹⁰L/mol **(****FIG. 4****).**

### ⑧ Detection of HLA-G isoform recognition specificity of antibodies by immunoblotting

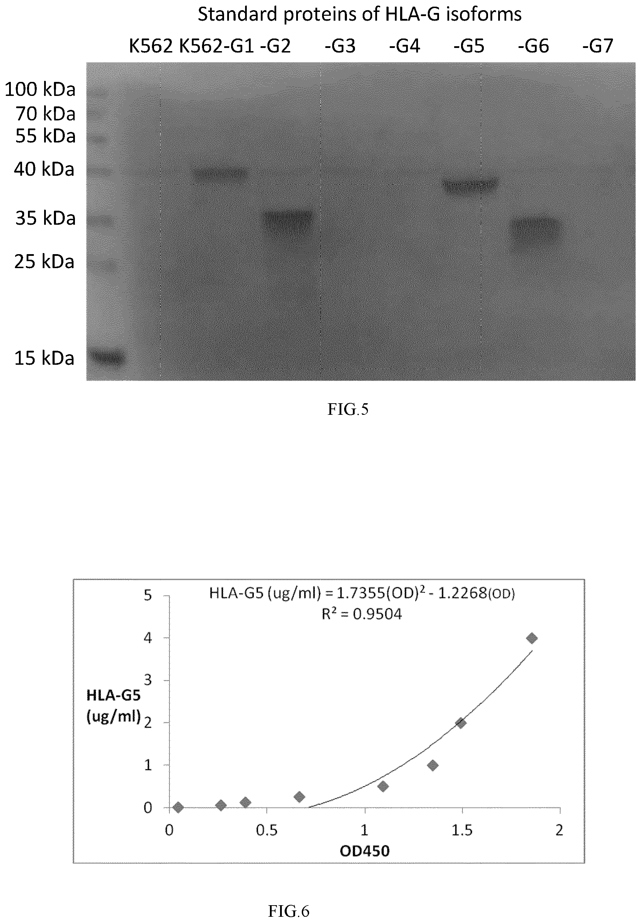

After electroporating the standard proteins of HLA-G isoforms (HLA-G1, HLA-G2, HLA-G3, HLA-G4, HLA-G5, HLA-G6 and HLA-G7), 5% skimmed milk powder was used to block at room temperature for 4 hours, and washed with 0.2% TBS (Tewen-20 PBS). Then the antibody (YWHG-3, 1.0 ug/ml) was added to detect its recognition specificity, incubated at 4°C overnight, and washed; and then HRP-labeled rabbit anti-mouse IgG antibody was added, incubated at room temperature for 30 minutes, and washed, then incubated with Dako REAL^{™} EnVision^{™} detection system (DAKO) for 1-3 minutes. The results showed that, the antibody (YWHG-3) can specifically recognize HLA-G1, -G2, -G5 and HLA-G6 standard proteins and has no cross-reaction with other HLA-G isoforms, and has good specificity for identifying HLA-G isoforms containing α3 domain (HLA-G1, -G2, -G5 and HLA-G6) **(****FIG. 5****).**

### II. Application examples

### Example 2.1. Detection of concentrations of HLA-G5 molecule of the antibody (YWHG-3) by ELISA

The HLA-G5 protein was diluted in a 2-fold gradient with a coating buffer (0.1M, pH=9.6 NaHCO3), to obtain the final concentrations of 4ug/ml, 2ug/ml, 1ug/ml, 0.5ug/ml, and 0.25ug/ml. 0.125ug/ml and 0.0625ug/ml respectively, with the coating buffer as a blank control. The plate was coated in 100 ul/well, and placed at 4°C overnight; then washed twice with the wash buffer; blocked with 1% BSA at 200ul/well, and incubated at 37°C for 2 hours; then washed 3 times with the wash buffer.

After washing, the biotin-labeled antibody (YWHG-3) with a final concentration of 1.0 ug/ml was added at 100 ul/well, and incubated at 37°C for 1 hour; then washed 3 times with the wash buffer. The peroxidase-conjugated Streptavidin diluted with 1.0 ug/ml PBS was added at100 ul/well, incubated at 37°C for 1 hour; taken out and washed 3 times. The color development was performed with TMB developing solution at 100ul/well for 5-15 minutes, and then 50 ul of stop solution was added to each well to stop. The absorbance valued was measured at the wavelength of 450, and a chart was drawn for analysis. The OD₄₅₀ values of the solutions at 4ug/ml, 2ug/ml, 1ug/ml, 0.5ug/ml, 0.25ug/ml, 0.125ug/ml, 0.0625ug/ml and the coating buffer blank control were 1.856,1.490, 1.347, 1.092,0.664, 0.388,0.263 and 0.045, respectively. The results showed that the antibody (YWHG-3) specifically recognized HLA-G5 and was significantly correlated with HLA-G5 concentration [HLA-G5 (ug/ml) =1.7355x (OD)² -1.2268x(OD); R² =0.9504] (FIG. 6).

### Example 2.2. Detection of HLA-G1, -G2, -G5 and HLA-G6 molecules of the antibody (YWHG-3) by flow cytometry

Cell cultures that expressed K562 (blank control), K562-HLA-G1, K562-HLA-G2, K562-HLA-G5 and K562-HLA-G6 in the logarithmic growth phase were taken and detected by intracellular flow cytometry.

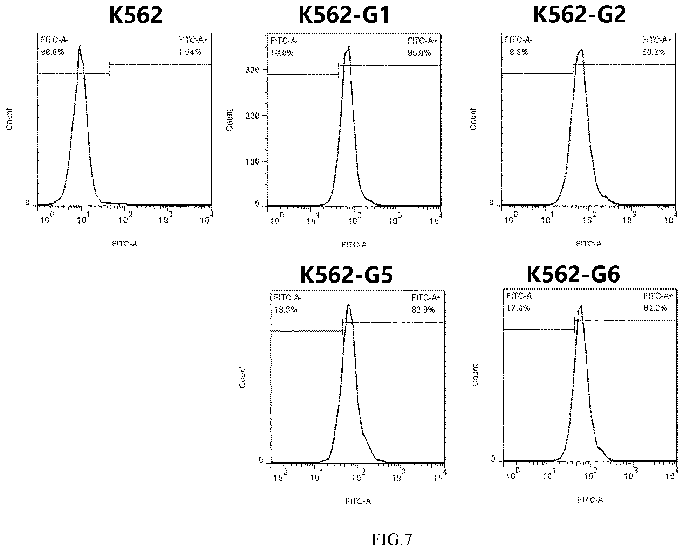

Flow cytometry for detection of intracellular molecular expression: the cell cultures of K562, K562-HLA-G1, K562-HLA-G2, K562-HLA-G5 and K562-HLA-G6 were collected in flow cytometric tubes, washed twice with 2% BSA/PBS (250 g), and 250 ul of cell membrane-breaking agent (BD Cytofix/Cytoperm^{™}) was added, and mixed thoroughly, then placed in the refrigerator at 4°C for 20 minutes. Then 1 ml BD Perm/Wash^{™} was added, centrifuged and washed twice (250 g). After washing, K562, K562-HLA-G1, K562-HLA-G2, K562-HLA-G5 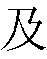 K562-HLA-G6 cells were resuspended with 100ul BD Perm/Wash^{™}, then 1 ul of 1.0 mg/ml FITC-labeled purified antibody (YWHG-3) was added, and incubated at 4°C for 30 minutes, centrifuged and washed with 1 ml of 1× BD Perm/Wash^{™} (250 g); then cells were resuspended to 300 ul of cell suspension with 1× BD Perm/Wash^{™} and detected by flow cytometry. The results showed that the antibody (YWHG-3) could specifically recognize intracellularly expressed K562-HLA-G1, K562-HLA-G2, K562-HLA-G5 and K562-HLA-G6 molecules (FIG. 7).

### Example 2.3.

### Use of the antibody (YWHG-3) for detection of HLA-G molecules (HLA-G1, HLA-G2, HLA-G5 and HLA-G6) in gastric cancer tissues by immunohistochemistry

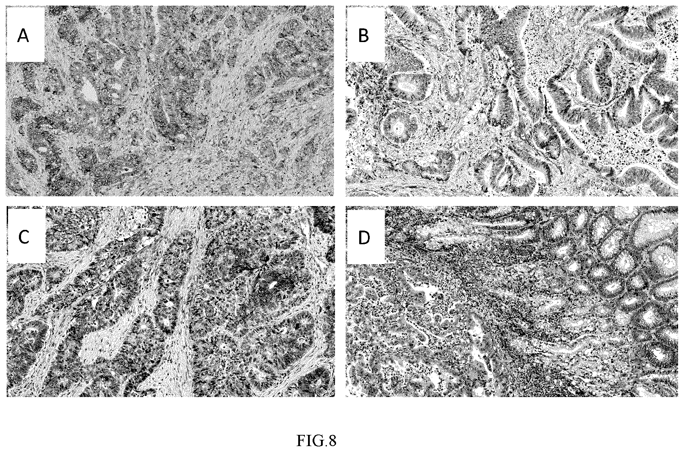

The gastric cancer tissues were fixed in 10%-12% neutral formalin and embedded in paraffin. Tissue sections underwent the conventional production processes such as baking, dewaxing, hydration and antigen retrieval. An appropriate amount of 1% BSA was added dropwise to the tissues to cover the tissue and tissue edges by 2 mm, and incubated at room temperature for 10 minutes for blocking. The antibody (YWHG-3) (1 mg/mL, 1:500 dilution) was added dropwise and placed in a humidified box at 4°C overnight (16-20 h), washed with TBS buffer, then the secondary antibody was added dropwise (goat anti-mouse ratio of the TBS diluted antibody at 1:300), and incubated in a 37°C incubator for 30 minutes. After washed with TBS buffer, the developing agent DAB working solution was added dropwise; when the color development of tissues was completed, the slides were washed with running water for 5 minutes, and soaked in distilled water for 5 minutes. After HE counterstaining, dehydration, transparency, and sealing, the visual field conditions of the tissue sections were observed under an optical microscope. The brown-colored cells indicated the positive expression of HLA-G isoforms (HLA-G1, HLA-G2, HLA-G5 and HLA-G6) in gastric cancer tissue cells. The expression intensity of HLA-G isoforms (HLA-G1, HLA-G2, HLA-G5 and HLA-G6) could be determined based on the brown coloring of cells. Among them, FIG. 8 showed high expression of HLA-G molecules in the gastric cancer tissue samples in (A), (B), (C) and (D) **(****FIG. 8****).**

## Claims

1. A monoclonal antibody (YWHG-3) against HLA-G1, -G2, -G5 and HLA-G6 isoforms, wherein the monoclonal antibody (YWHG-3) is produced by the hybridoma deposited under CCTCC NO: 202121, the depositary institution is China Center for Type Culture Collection and the depositary time is August 11, 2021.

2. A monoclonal antibody (YWHG-3) against HLA-G1, -G2, -G5 and HLA-G6 isoforms, wherein the monoclonal antibody (YWHG-3) at least comprises one or more of light chain hypervariable regions CDR1, CDR2 and CDR3, and/or one or more of heavy chain hypervariable regions CDR1, CDR2 and CDR3;
an amino acid sequence of the antibody light chain of the monoclonal antibody (YWHG-3) is shown in SEQ ID No. 1 or an amino acid sequence with equivalent functions to the sequence shown in SEQ ID No. 1 formed by substituting, deleting or adding one or more amino acids; an amino acid sequence of the hypervariable region CDR1 of the antibody light chain is the sequence QSLVHSTGNTF shown in SEQ ID No. 2 or an amino acid sequence with equivalent functions to the sequence shown in SEQID No. 2 formed by substituting, deleting or adding one or more amino acids; an amino acid sequence of the hypervariable region CDR2 of the light chain is the sequence KVS shown in SEQ ID No. 3 or an amino acid sequence with equivalent functions to the sequence shown in SEQ ID No. 3 formed by substituting, deleting or adding one or more amino acids, and an amino acid sequence of the hypervariable region CDR3 of the light chain is the sequence SQSTHVPWT shown in SEQ ID No. 4 or an amino acid sequence with equivalent functions to the sequence shown in SEQ ID No. 4 formed by substituting, deleting or adding one or more amino acids;
an amino acid sequence of the antibody heavy chain of the monoclonal antibody (YWHG-3) is shown in SEQ ID No. 5 or an amino acid sequence with equivalent functions to the sequence shown in SEQ ID No. 5 formed by substituting, deleting or adding one or more amino acids; an amino acid sequence of the hypervariable region CDR1 of the antibody heavy chain is the sequence GFTFSDYN shown in SEQ ID No. 6 or an amino acid sequence with equivalent functions to the sequence shown in SEQID No. 6 formed by substituting, deleting or adding one or more amino acids; an amino acid sequence of the hypervariable region CDR2 of the heavy chain is the sequence ISDGGRST shown in SEQ ID No. 7 or an amino acid sequence with equivalent functions to the sequence shown in SEQ ID No. 7 formed by substituting, deleting or adding one or more amino acids, and an amino acid sequence of the hypervariable region CDR3 of the heavy chain is the sequence VRDDY shown in SEQ ID No. 8 or an amino acid sequence with equivalent functions to the sequence shown in SEQ ID No. 8 formed by substituting, deleting or adding one or more amino acids.

3. The monoclonal antibody (YWHG-3) against HLA-G1, -G2, -G5 and HLA-G6 isoforms according to claim 2, wherein the monoclonal antibody (YWHG-3) further comprises a light chain framework region (FR) and a heavy chain framework region; wherein, the light chain framework region comprises one or more of light chains FR1, FR2, FR3 and FR4, and an amino acid sequence of the light chain FR1 is the sequence DIMMTQTPLSLPVSLGDQASISCRSS shown in SEQ ID No. 9 or an amino acid sequence with equivalent functions to the sequence shown in SEQ ID No. 9 formed by substituting, deleting or adding one or more amino acids; an amino acid sequence of the light chain FR2 is the sequence LNWYLQKPGQSPKLLIY shown in SEQ ID No. 10 or an amino acid sequence with equivalent functions to the sequence shown in SEQ ID No. 10 formed by substituting, deleting or adding one or more amino acids; an amino acid sequence of the light chain FR3 is the sequence NRFSGVSDRFSGSGSGTDFTLKIRRVEAEDLGVYFC shown in SEQ ID No. 11 or an amino acid sequence with equivalent functions to the sequence shown in SEQ ID No. 11 formed by substituting, deleting or adding one or more amino acids; an amino acid sequence of the light chain FR4 is the sequence FGGGTKLEIK shown in SEQ ID No. 12 or an amino acid sequence with equivalent functions to the sequence shown in SEQ ID No. 12 formed by substituting, deleting or adding one or more amino acids;
the heavy chain framework region comprises one or more of the heavy chains FR1, FR2, FR3 and FR4, wherein, an amino acid sequence of the heavy chain FR1 is the sequence LVQLQQSGGGLVKPGGSLKLSCAAS shown in SEQ ID No. 13 or an amino acid sequence with equivalent functions to the sequence shown in SEQ ID No. 13 formed by substituting, deleting or adding one or more amino acids; an amino acid sequence of the heavy chain FR2 is the sequence MYWVRQTPEKRLEWVAT shown in SEQ ID No. 14 or an amino acid sequence with equivalent functions to the sequence shown in SEQ ID No. 14 formed by substituting, deleting or adding one or more amino acids; an amino acid sequence of the heavy chain FR3 is the sequence FYLDSVKGRFTISRDNAKNNLYLQINNLKSEDTAIYYC shown in SEQ ID No. 15 or an amino acid sequence with equivalent functions to the sequence shown in SEQ ID No. 15 formed by substituting, deleting or adding one or more amino acids; an amino acid sequence of the heavy chain FR4 is the sequence WGQGTTLTVSS shown in SEQ ID No. 16 or an amino acid sequence with equivalent functions to the sequence shown in SEQ ID No. 16 formed by substituting, deleting or adding one or more amino acids.

4. The monoclonal antibody (YWHG-3) against HLA-G1, -G2, -G5 and HLA-G6isoforms according to claim 2, wherein a nucleotide sequence encoding the light chain variable region in the monoclonal antibody (YWHG-3) is a sequence shown in SEQ ID No. 17 or a nucleotide sequence with equivalent functions to the sequence shown in SEQ ID No. 17 formed by substituting, deleting or adding one or more nucleotides; a nucleotide sequence encoding the heavy chain variable region in the monoclonal antibody (YWHG-3) is a sequence shown in SEQ ID No. 18 or a nucleotide sequence with equivalent functions to the sequence shown in SEQ ID No. 18 formed by substituting, deleting or adding one or more nucleotides.

5. Use of the monoclonal antibody (YWHG-3) against HLA-G1, -G2, -G5 and HLA-G6 isoforms of any one of claims 1 to 4 for immunohistochemistry, immunoblotting and intracellular flow cytometry.
